# NOUVEAU FASCICULE DE BREVET EUROPEEN

(11) **EP 0 351 297 B2**
(45) Date de publication et mention de la décision concernant l'opposition: **05.03.1997**
(45) Mention de la délivrance du brevet: 08.07.1992
(21) Numéro de dépôt: 89401973.6
(22) Date de dépôt: 10.07.1989
(51) Int. Cl.: A61K 7/06

(54) **Composition cosmétique capillaire à base de polyorganosiloxanes à fonction hydroxyalkyle**
Haarkosmetisches Präparat mit Polyorganosiloxanen, die Hydroxyalkylgruppen tragen
Hair-cosmetic compound based on polyorganosiloxanes containing hydroxyalkyl groups

(30) Priorité: 12.07.1988 LU 87273
(43) Date de publication de la demande: 17.01.1990
(73) Titulaire: L'OREAL, 75008 Paris (FR)
(72) Inventeur: Grollier, Jean-François, F-75004 Paris (FR); Dubief, Claude, F-78150 Le Chesnay (FR); Dupuis, Christine, F-75018 Paris (FR)
(74) Mandataire: Casalonga, Axel

(56) Documents cités:
- EP-A- 0 057 837
- DE-A- 1 617 388
- DE-A- 3 041 141
- DE-A- 3 206 448
- FR-A- 2 443 476
- GB-A- 2 173 515
- Chem. Abstract vol. 105 Nr 213935q, 1986
- Chem. Abstract vol. 94 Nr 162601q, 1981

## Description

La présente invention a pour objet une composition cosmétique capillaire sous forme de lotion à base de polyorganoslioxanes à fonction hydroxyalkyle, ainsi qu'aux procédés de traitement des cheveux avec ladite composition.

Certains polyorganosiloxanes sont bien connus dans le domaine de la cosmétique capillaire et ont été utilisés comme agents de conditionnement de la chevelure, recherchés notamment pour obtenir une chevelure brillante. Il s'agit essentiellement de phénylméthylpolysiloxanes présents dans des compositions cosmétiques que l'on applique en général sur la chevelure sans faire suivre cette application d'un rinçage.

Ces composés présentent cependant l'inconvénient d'alourdir les cheveux. Esthétiquement on recherche pour la coiffure des propriétés de brillance associées à un aspect gonflant et léger.

La demanderesse a découvert de façon surprenante que l'utilisation de polyorganosiloxanes à fonction hydroxyalkyle conférait à la chevelure un aspect brillant et permettait d'obtenir une chevelure légère et gonflante , recherchée en cosmétique.

On appelle "traitement cosmétique" un traitement visant à obtenir sur les cheveux un ou plusieurs des résultats indiqués ci-dessus.

Un objet de l'invention est constitué par les compositions cosmétiques destinées au traitement des cheveux mettant en oeuvre les polyorganosloxanes à fonction hydroxyalkyle et ne contenant pas d'agents tensio-actifs anioniques dans des proportions conférant à la composition des propriétés moussantes.

L'invention a également pour objet un procédé de traitement cosmétique des cheveux mettant en oeuvre ces compositions, comme produits coiffants.

D'autres objets de l'invention apparaîtront à la lecture de la description et des exemples qui suivent.

La présente invention a donc pour objet principal une composition cosmétique capillaire sous forme de lotion contenant dans un milieu cosmétiquement acceptable au moins un polyorganosiloxane à fonction hydroxyalkyle répondant à la formule suivante (I) : 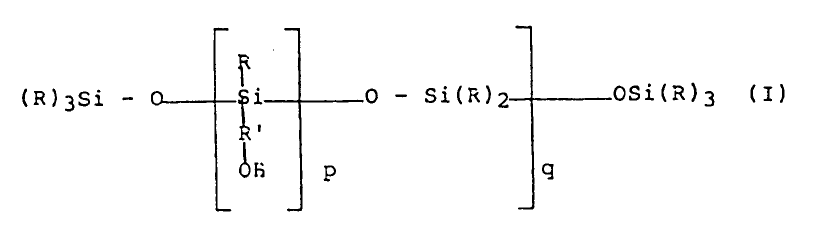 dans laquelle les radicaux R, identiques ou différents, sont choisis parmi les radicaux méthyle et phényle, 60% molaire au moins des radicaux R étant des radicaux méthyle, le radical R' est un chaînon alkylène divalent hydrocarboné, linéaire ou ramifié, comportant de 2 à 18 atomes de carbone;
p est un nombre entier compris entre 1 et 30 inclus, de préférence entre 1 et 20 inclus;
q est un nombre entier compris entre 1 et 120 inclus, dans des concentrations comprises entre 0,5 et 50% en poids par rapport au poids total de la composition et de 50 à 99,5% en poids par rapport au poids total de la composition d'un solvant choisi parmi les alcanols inférieurs, les silicones volatiles ou les hydrocarbures choisis parmi le butane et les hydrocarbures fluorocarbonés ou leurs mélanges ;
cette composition ne contenant pas d'agents tensio-actifs anioniques dans des proportions conférant à la composition des propriétés moussantes.

Le copolymère selon l'invention peut être un copolymère alterné ou statistique.

Parmi les composés particulièrement préférés utilisés conformément à l'invention, on peut citer des composés de formule (I) dans laquelle :
R' représente un chaînon alkylène divalent hydrocarboné, linéaire ou ramifié, comportant de 2 à 6 atomes de carbone et plus particulièrement, une chaîne triméthylène :

   -(CH₂)̵₃

   ou un chaînon méthyl-2 triméthylène :

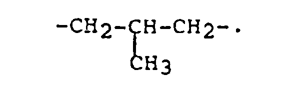

Parmi les produits de formule (I), on préfère utiliser des produits dont la masse moléculaire en nombre est comprise entre 600 et 10.000.

Des produits de formule (I) plus particulièrement préférés sont représentés par les produits de masse moléculaire en nombre compris entre 1000 et 10.000, pour lesquels p est compris entre 1 et 5 et q est compris entre 10 et 100.

Les produits de formule (I) sont connus dans l'industrie et peuvent être préparés suivant les procédés connus de l'art antérieur, tels que ceux décrits dans le brevet francais n° 85 16.334.

Pour préparer les produits de formule (I), on peut par exemple utiliser comme organopolysiloxane de départ le copolymère de formule (II) : 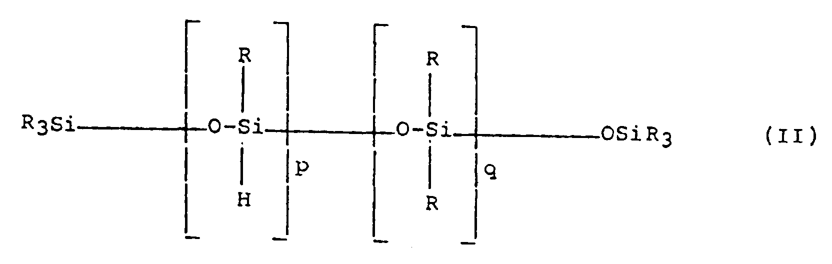 dans laquelle R, p et q ont la signfication donnée ci-dessus.

Ces produits sont bien connus dans l'industrie des silicones et sont généralement disponibles dans le commerce. Ils sont par exemple décrits dans les brevets US-A-3 220 942, 3 341 111 et 3 436 366.

Sur les produits de formule (II), on fait réagir un alcool à insaturation alcénique de formule (III) : R''OH, dans laquelle R'' est un radical alcènylène linéaire ou ramifié ayant de 2 à 18 atomes de carbone. Parmi ces alcools, on utilise plus particulièrement l'alcool allylique et l'alcool méthallylique.

On peut utiliser comme catalyseur d'hydrosilylation pour faire réagir les alcools insaturés de formule (III) sur l'hydrogénopolysiloxane de formule (II), des catalyseurs d'hydrosilylation connus, notamment les complexes du platine décrits dans les brevets US-A-3 715 334, 3775 452 et 3 814 730; les complexes platine oléfine décrits dans les brevets US-A-3 159 601 et 3 159 662.

Des procédés de préparation des produits de formule (I) sont décrits en détail dans les brevets US-A-2 970 150 et 4 160 775.

Les compositions cosmétiques pour le traitement et le soin des cheveux conformes à l'invention contiennent dans un milieu cosmétiquement acceptable, un polyorganosiloxane à fonction hydroxyalkyle de formule (I) dans des concentrations comprises de préférence entre 1 et 30% par rapport au poids total de la composition.

Ces compositions peuvent se présenter sous forme de lotions aqueuses, alcooliques ou à base de solvants organiques, épaissies ou non, ou conditionnées en aérosol ou en flacon pompe pour former des sprays.

Elles peuvent contenir en plus du polyorganosiloxane de formule (I), des adjuvants habituellement utilisés en cosmétique, tels que des parfums, des colorants, des conservateurs, des épaississants, des agents tensio-actifs non ioniques, cationiques, amphotères ou leur mélange, des filtres solaires, ainsi que des substances actives.

Cette composition ne contient pas d'agent tensio-actif anionique dans des proportions conférant à la composition des propriétés moussantes.

Cette proportion est inférieure à 5% et de préférence inférieure à 3% en poids par rapport au poids total de la composition, celle-ci ne contenant pas, de préférence, d'agent tensio-actif anionique.

Les compositions cosmétiques destinées au traitement des cheveux, conformes à l'invention, sont utilisées de préférence dans des applications non suivies d'un rinçage. Elles peuvent être utilisées en particulier comme produits coiffants non rincés, tels que dans des lotions de mise en plis ou brushing ou encore dans les laques.

Lorsque les compositions cosmétiques non rincées, selon l'invention, sont sous forme de lotions, le milieu solvant peut être choisi parmi les alcools inférieurs en C₂ à C₄ et de préférence parmi l'alcool éthylique ou parmi les silicones volatiles telles que les silicones cycliques décrites dans le dictionnaire CTFA sous les appellations HEXAMETHYLSILOXANES et CYCLOMETHICONES ou leurs mélanges, ou encore parmi les hydrocarbures tels que de préférence le butane et les hydrocarbures fluorochlorés, choisis de préférence parmi le fluorotrichlorométhane et le difluorochlorométhane.

Les solvants sont présents dans ces compositions non rincées à des concentrations comprises de préférence entre 70 et 98% par rapport au poids total de la composition.

Lorsque les compositions sont épaissies, elles contiennent un ou plusieurs épaississants pouvant être choisis de préférence parmi les dérivés cellulosiques, tels que la méthylcellulose, l'hydroxyméthylcellulose, l'hydroxyéthylcellulose, l'hydroxypropylcellulose, l'hydroxypropylméthylcellulose, la carboxyméthylcellulose, la gomme de guar ou ses dérivés, les polymères d'acide acrylique, réticulés ou non, les copolymères éthylène/anhydride maléïque, les copolymères méthylvinyléther/anhydride maléïque, la gomme de xanthane et les scléroglucanes.

On peut épaissir la composition en utilisant le produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol, à une concentration de 5% et à 30°C, inférieure ou égale à 30.10⁻³ Pa.s, tel que décrit plus particulièrement dans la demande de brevet français n° 2 598 611.

Les épaississants particulièrement préférés pour épaissir les compositions non rincées sont choisis parmi les polymères d'acide acrylique réticulés ou non et plus particulièrement les acides polyacryliques réticulés par un agent polyfonctionnel, tels que les produits vendus sous la dénomination CARBOPOL par la Société GOODRICH, les dérivés de cellulose tels qu'indiqués ci-dessus, les copolymères éthylène anhydride maléique, tels que ceux vendus par la Société MONSANTO sous la dénomination EMA 91, les copolymères de méthylvinyléther et d'anhydride maléique, tels que ceux vendus par la Société GAF sous la dénomination GANTREZ AN (119, 139, 169) et les produits résultant de l'interaction ionique de polymères tels que ceux décrits ci-dessus.

La concentration en agent épaississant dans ces compositions varie entre 0,05 et 5% et de préférence entre 0,1 et 2% en poids par rapport au poids total de la composition.

Les compositions conformes à l'invention peuvent être conditionnées en aérosol pour être distribuées sous forme de sprays et former des laques. On utilise dans ce cas la composition en présence d'un gaz propulseur, tel que plus particulièrement le gaz carbonique, l'azote, le protoxyde d'azote, l'éther diméthylique, les hydrocarbures volatils comme le butane, l'isobutane, le propane, les hydrocarbures chlorés et/ou fluorés, les mélanges d'hydrocarbures tels que le n-butane, l'isobutane, le propane, avec des hydrocarbures chlorofluorés. Dans les compositions non rincées, on choisit de préférence les alcanes, les fluoroalcanes, les chlorofluoroalcanes et leurs mélanges dans des proportions ne dépassant pas 80% par rapport au poids total de la composition.

Le procédé de traitement cosmétique mettant en oeuvre les polyorganosiloxanes de formule (I) définis ci-dessus, consiste essentiellement à appliquer la composition sur les cheveux, cette application étant éventuellement suivie d'un rinçage, en vue d'en améliorer la brillance, tout en leur conférant un aspect léger et gonflant.

Les exemples suivants sont destinés à illustrer l'invention sans pour autant présenter un caractère limitatif.

### EXEMPLE 1

On prépare un spray capillaire aérosol de composition suivante :

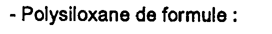

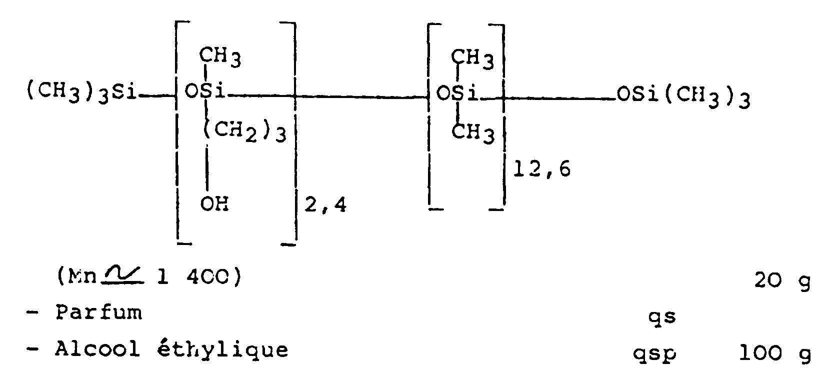

### Conditionnement aérosol :

| | |
|---|---|
| - Composition ci-dessus | 30 g |
| - Fluorotrichlorométhane/difluorochlorométhane 60/40 | 60 g |
| - Mélange ternaire de N-butane, isobutane > 55%, propane, vendu sous la dénomination AEROGAZ 3,2 N par la Société ELF AQUITAINE | 10 g |

### EXEMPLE 2

On prépare un spray capillaire de composition suivante :

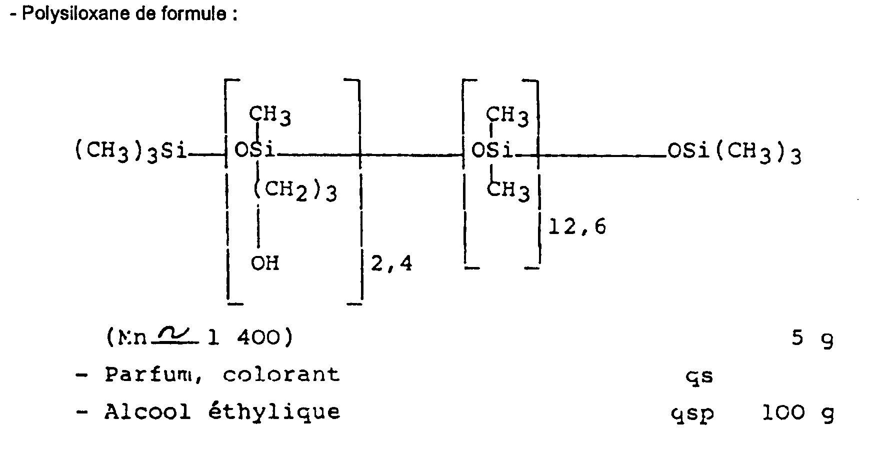

On conditionne ce spray en flacon pompe.

### EXEMPLE 4

On prépare un spray capillaire de composition suivante :

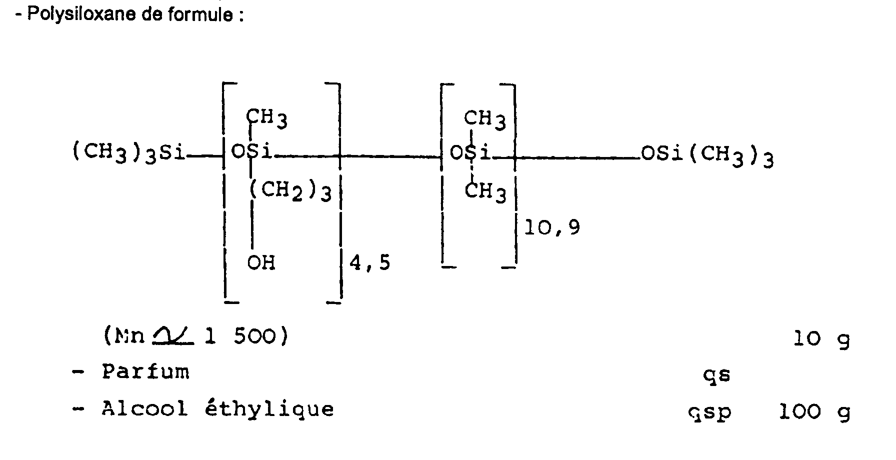

que l'on conditionne en flacon pompe.

### EXEMPLE 4

On prépare un spray de brillance de composition suivante :

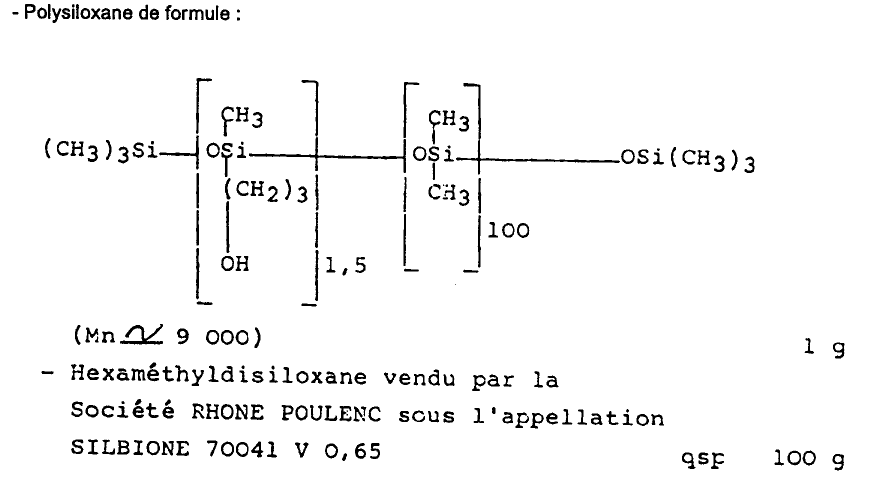

Cette composition est conditionnée en flacon pompe et pulvérisée sur cheveux secs.

Les cheveux sont légers et plus brillants que lorsqu'ils sont traités avec une composition similaire à 1% de phénylméthylpolysiloxane.

## Revendications (Revendications pour l'(les) Etat(s) contractant(s) suivant(s): AT, BE, CH, DE, FR, GB, GR, IT, LU, NL, SE)

1. Composition cosmétique capillaire caractérisée par le fait qu'elle se présente sous forme de lotion, contenant au moins :
- de 0,5 à 50% en poids par rapport au poids total de la composition d'un polyorganosiloxane à fonction hydroxyalkyle, répondant à la formule (I) suivante : 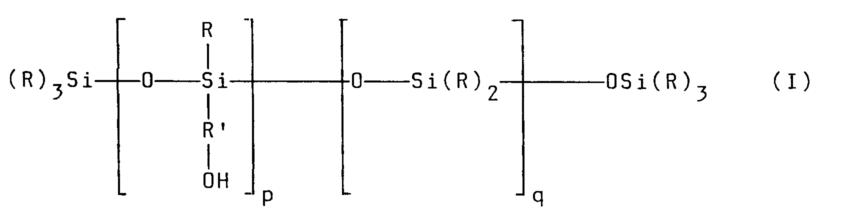 dans laquelle les radicaux R, identiques ou différents, sont choisis parmi les radicaux méthyle et phényle, 60% molaire au moins des radicaux R étant des radicaux méthyle, le radical R' est un chaînon alkylène divalent hydrocarboné, linéaire ou ramifié, comportant de 2 à 18 atomes de carbone ; p est un nombre entier compris entre 1 et 30 inclus ; q est un nombre entier compris entre 1 et 120 inclus ;
- de 50 à 99,5% en poids par rapport au poids total de la composition, d'un solvant choisi parmi les alcanols inférieurs en C₂-C₄, les silicones volatiles ou les hydrocarbures choisis parmi le butane et les hydrocarbures fluorochlorés ou leurs mélanges ;
cette composition ne contenant pas d'agents tensio-actifs anioniques dans des proportions suffisantes pour conférer à la composition des propriétés moussantes.

2. Composition selon la revendication 1, caractérisée par le fait que dans les composés de formule (I), R' représente un chaînon alkylène divalent hydrocarboné, linéaire ou ramifié, comportant de 2 à 6 atomes de carbone.

3. Composition selon la revendication 1 ou 2, caractérisée par le fait que dans les composés de formule (I), R' est le radical triméthylène : -(CH₂)̵₃ ou un chainon méthyl-2 triméthylène :

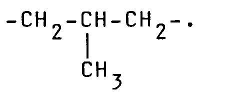

4. Composition selon l'une quelconque des revendications 1 à 3, caractérisée par le fait qu'elle est conditionnée en aérosol en présence d'un gaz propulseur, pour former au moment de l'expulsion un spray.

5. Utilisation de la composition telle que définie dans l'une quelconque des revendications 1 à 4, comme produit coiffant, sans mettre en oeuvre un rinçage.

6. Procédé de traitement cosmétique des cheveux, caractérisé par le fait que l'on applique sur ceux-ci au moins une composition telle que définie dans l'une quelconque des revendications 1 à 4, sans que cette application soit suivie d'un rinçage.

## Revendications (Revendications pour l'(les) Etat(s) contractant(s) suivant(s): ES)

1. Composition cosmétique capillaire caractérisée par le fait qu'elle se présente sous forme de lotion, contenant au moins :
- de 0,5 à 50% en poids par rapport au poids total de la composition d'un polyorganosiloxane à fonction hydroxyalkyle, répondant à la formule (I) suivante : 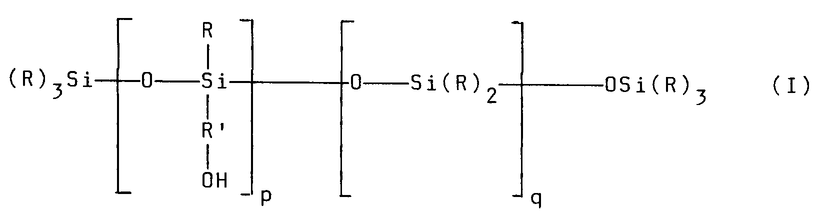 dans laquelle les radicaux R, identiques ou différents, sont choisis parmi les radicaux méthyle et phényle, 60% molaire au moins des radicaux R étant des radicaux méthyle, le radical R' est un chaînon alkylène divalent hydrocarboné, linéaire ou ramifié, comportant de 2 à 18 atomes de carbone ; p est un nombre entier compris entre 1 et 30 inclus ; q est un nombre entier compris entre 1 et 120 inclus ;
- de 50 à 99,5% en poids par rapport au poids total de la composition, d'un solvant choisi parmi les alcanols inférieurs en C₂-C₄, les silicones volatiles ou les hydrocarbures choisis parmi le butane et les hydrocarbures fluorochlorés ou leurs mélanges ;
cette composition ne contenant pas d'agents tensio-actifs anioniques dans des proportions suffisantes pour conférer à la composition des propriétés moussantes.

2. Composition selon la revendication 1, caractérisée par le fait que dans les composés de formule (I), R' représente un chaînon alkylène divalent hydrocarboné, linéaire ou ramifié, comportant de 2 à 6 atomes de carbone.

3. Composition selon la revendication 1 ou 2, caractérisée par le fait que dans les composés de formule (I), R' est le radical triméthylène : -(CH₂)̵₃ ou un chainon méthyl-2 triméthylène :

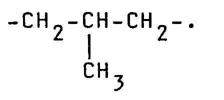

4. Composition selon l'une quelconque des revendications 1 à 3, caractérisée par le fait qu'elle est conditionnée en aérosol en présence d'un gaz propulseur, pour former au moment de l'expulsion un spray.

## Claims (Claims for the following Contracting State(s): AT, BE, CH, DE, FR, GB, GR, IT, LU, NL, SE)

1. Cosmetic hair composition characterised in that it is in the form of a lotion containing at least: - from 0.5 to 50 % by weight, relative to the total weight of the composition, of a polyorganosiloxane containing a hydroxyalkyl functional group of the following formula (I): 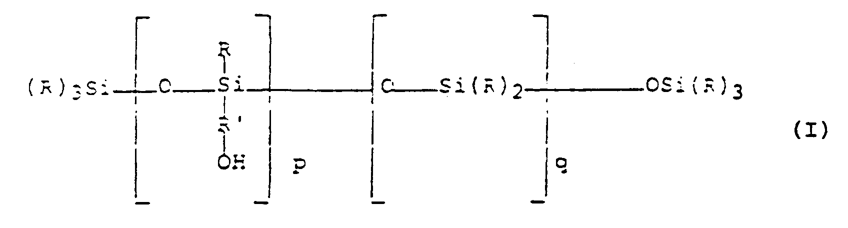 in which the radicals R, which are identical or different, are chosen from methyl and phenyl radicals, at least 60 mole % of the radicals R being methyl radicals, the radical R' is a linear or branched divalent hydrocarbon alkylene group containing 2 to 18 carbon atoms; p is an integer between 1 and 30 inclusive; q is an integer between 1 and 120 inclusive;
- 50 to 99.5 % by weight, relative to the total weight of the composition, of a solvent chosen from lower C₂-C₄ alkanols, volatile silicones or hydrocarbons chosen from butane and chlorofluorocarbons or mixtures thereof;
this composition not containing anionic surface-active agents in sufficient proportions to confer foaming properties on the composition.

2. Composition according to Claim 1, characterised in that in the compounds of formula (I), R' represents a linear or branched divalent hydrocarbon alkylene group containing 2 to 6 carbon atoms.

3. Composition according to Claim 1 or 2, characterised in that in the compounds of formula (I), R' is a trimethylene radical: -(CH₂)₃- or a 2-methyltrimethylene group:

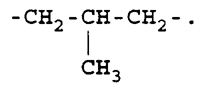

4. Composition according to any one of Claims 1 to 3, characterised in that it is packaged as an aerosol in the presence of a propelling gas, to form a spray at the time of expulsion.

5. Use of the composition as defined in any one of Claims 1 to 4, as a hair-styling product, without the need for rinsing.

6. Method for the cosmetic treatment of hair, characterised in that at least one composition as defined in any one of Claims 1 to 4 is applied to it, without this application being followed by rinsing.

## Claims (Claims for the following Contracting State(s): ES)

1. Cosmetic hair composition characterised in that it is in the form of a lotion containing at least: - from 0.5 to 50 % by weight, relative to the total weight of the composition, of a polyorganosiloxane containing a hydroxyalkyl functional group of the following formula (I): 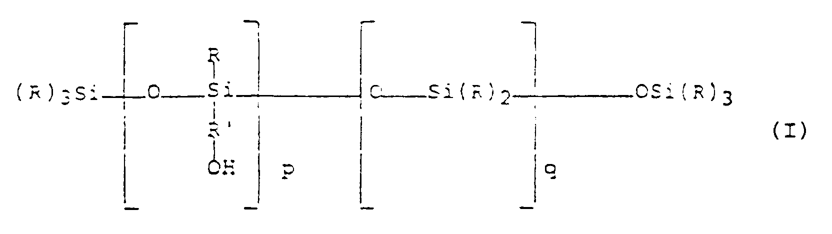 in which the radicals R, which are identical or different, are chosen from methyl and phenyl radicals, at least 60 mole % of the radicals R being methyl radicals, the radical R' is a linear or branched divalent hydrocarbon alkylene group containing 2 to 18 carbon atoms; p is an integer between 1 and 30 inclusive; q is an integer between 1 and 120 inclusive;
- 50 to 99.5 % by weight, relative to the total weight of the composition, of a solvent chosen from lower C₂-C₄ alkanols, volatile silicones or hydrocarbons chosen from butane and chlorofluorocarbons or mixtures thereof;
this composition not containing anionic surface-active agents in sufficient proportions to confer foaming properties on the composition.

2. Composition according to Claim 1, characterised in that in the compounds of formula (I), R' represents a linear or branched divalent hydrocarbon alkylene group containing 2 to 6 carbon atoms.

3. Composition according to Claim 1 or 2, characterised in that in the compounds of formula (I), R' is a trimethylene radical: -(CH₂)₃- or a 2-methyltrimethylene group:

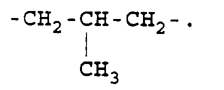

4. Composition according to any one of Claims 1 to 3, characterised in that it is packaged as an aerosol in the presence of a propelling gas, to form a spray at the time of expulsion.

## Patentansprüche (Patentansprüche für folgende(n) Vertragsstaat(en): AT, BE, CH, DE, FR, GB, GR, IT, LI, NL, SE)

1. Haarkosmetische Zusammensetzung, dadurch gekennzeichnet, dass sie in Form einer Lotion vorliegt, enthaltend mindestens:
- 0,5 bis 50 Gew.%, bezogen auf das Gesamtgewicht der Zusammensetzung, eines Polyorganosiloxans mit Hydroxyalkylfunktion der folgenden Formel (I): 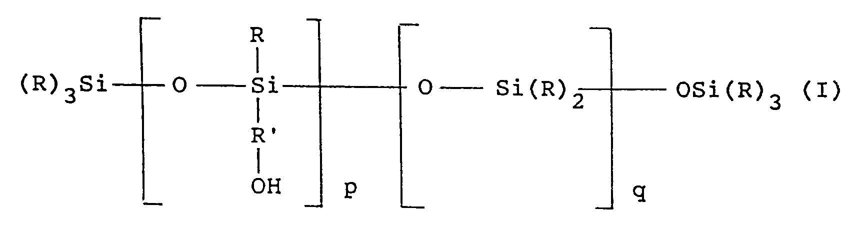 worin die Reste R, gleich oder verschieden, unter Methyl- und Phenylresten ausgewählt sind, wobei mindestens 60 Mol-% der Reste R Methylreste sind, und worin der Rest R' ein lineares oder verzweigtes, divalentes Alkylenkettenglied mit 2 bis 18 Kohlenstoffatomen, p eine ganze Zahl von 1 bis 30 und q eine ganze Zahl von 1 bis 120 sind;
- 50 bis 99,5 Gew.% , bezogen auf das Gesamtgewicht der Zusammensetzung, eines Lösungsmittels, ausgewählt aus C₂₋₄-Niedrigalkanolen, flüchtigen Siliconen oder aus Kohlenwasserstoffen, ausgewählt aus Butan und Fluorkohlenwasserstoffen, oder aus deren Mischungen,
wobei diese Zusammensetzung keine anionischen oberflächenaktiven Mittel in hinreichenden Mengen enthält, um der Zusammensetzung schäumende Eigenschaften zu verleihen.

2. Zusammensetzung gemäss Anspruch 1, dadurch gekennzeichnet, dass in den Verbindungen der Formel (I) R' ein lineares oder verzweigtes, divalentes Alkylenkettenglied mit 2 bis 6 Kohlenstoffatomen darstellt.

3. Zusammensetzung gemäss Anspruch 1 oder 2, dadurch gekennzeichnet, dass in den Verbindungen der Formel (I) R' der Trimethylenrest -(CH₂)₃- oder ein 2-Methyltrimethylen-Kettenglied 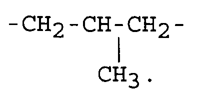 ist

4. Zusammensetzung gemäß einem der Ansprüche 1 bis 3, dadurch gekennzeichnet, daß
sie als Aerosol in Gegenwart eines gasförmigen Treibmittels zubereitet ist, um zum Zeitpunkt der Ausbringung ein Spray zu bilden.

5. Verwendung der in einem der Ansprüche 1 bis 4 definierten Zusammensetzung als Produkt zum Frisieren, ohne eine Spülung durchzuführen.

6. Verfahren zur kosmetischen Behandlung der Haare, dadurch gekennzeichnet, daß man auf diese mindestens eine in einem der Ansprüche 1 bis 4 definierte Zusammensetzung aufbringt, ohne daß danach gespült wird.

## Patentansprüche (Patentansprüche für folgende(n) Vertragsstaat(en): ES)

1. Haarkosmetische Zusammensetzung, dadurch gekennzeichnet, dass sie in Form einer Lotion vorliegt, enthaltend mindestens:
- 0,5 bis 50 Gew.%, bezogen auf das Gesamtgewicht der Zusammensetzung, eines Polyorganosiloxans mit Hydroxyalkylfunktion der folgenden Formel (I): 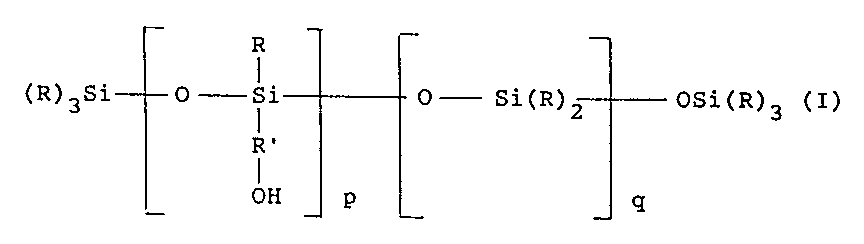 worin die Reste R, gleich oder verschieden, unter Methyl- und Phenylresten ausgewählt sind, wobei mindestens 60 Mol-% der Reste R Methylreste sind, und worin der Rest R' ein lineares oder verzweigtes, divalentes Alkylenkettenglied mit 2 bis 18 Kohlenstoffatomen, p eine ganze Zahl von 1 bis 30 und q eine ganze Zahl von 1 bis 120 sind;
- 50 bis 99,5 Gew.% , bezogen auf das Gesamtgewicht der Zusammensetzung, eines Lösungsmittels, ausgewählt aus C₂₋₄-Niedrigalkanolen, flüchtigen Siliconen oder aus Kohlenwasserstoffen, ausgewählt aus Butan und Fluorkohlenwasserstoffen, oder aus deren Mischungen,
wobei diese Zusammensetzung keine anionischen oberflächenaktiven Mittel in hinreichenden Mengen enthält, um der Zusammensetzung schäumende Eigenschaften zu verleihen.

2. Zusammensetzung gemäss Anspruch 1, dadurch gekennzeichnet, dass in den Verbindungen der Formel (I) R' ein lineares oder verzweigtes, divalentes Alkylenkettenglied mit 2 bis 6 Kohlenstoffatomen darstellt.

3. Zusammensetzung gemäss Anspruch 1 oder 2, dadurch gekennzeichnet, dass in den Verbindungen der Formel (I) R' der Trimethylenrest -(CH₂)₃- oder ein 2-Methyltrimethylen-Kettenglied 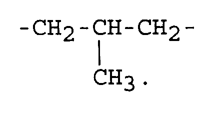 ist

4. Zusammensetzung gemäß einem der Ansprüche 1 bis 3, dadurch gekennzeichnet, daß
sie als Aerosol in Gegenwart eines gasförmigen Treibmittels zubereitet ist, um zum Zeitpunkt der Ausbringung ein Spray zu bilden.
